# EUROPEAN PATENT APPLICATION

(11) **EP 4 793 962 A1**
(43) Date of publication of application: **19.08.2026**
(21) Application number: 23955147.6
(22) Date of filing: 12.10.2023
(51) Int. Cl.: G16H 20/60, A61B 5/145, H04W 4/02, G08B 21/24

(54) **METHOD FOR SEARCHING FOR LOST DEVICE, APPLIED TO DIABETES MANAGEMENT SYSTEM**

(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2023/124254
(87) International publication number: WO 2025/076766

(57) **Abstract**

The invention discloses a method of locating a lost device in a diabetes management system, the diabetes management system comprises at least one wearable medical device; at least one personal management device and a secondary management device, at least one of the personal management device and the secondary management device is configured to control the wearable medical device, the personal management device and the secondary management device are communicative connection with each other; when either the personal management device or the secondary management device is determined as lost, the other device is used to locate the lost one, thereby enabling the user to retrieve it with ease.

## Description

### TECHNICAL FIELD

The present invention mainly relates to the field of medical device, and in particular to a method of locating a lost device and a method of preventing the device loss in a diabetes management system.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods of continuously monitoring blood glucose are based on in vivo glucose monitoring devices, which use disposable transdermal sensors inserted into the skin to measure glucose concentrations in interstitial fluids and send the blood glucose data through a transmitter to the remote device in real-time for the user to view. This monitoring method is called Continuous Glucose Monitoring (CGM).

Depending on the severity of the disease and the health status of the individual patient, the doctor will give different recommendations, for example, for patients with very mild symptoms, it may only be necessary to measure a small amount of fingertip blood for testing on a regular basis. Diabetic patients with mild symptoms can complete self-monitoring and management of blood glucose through continuous glucose monitoring, while diabetic patients with more severe symptoms need to be treated by the doctor in the hospital, such as adopting targeted treatment plans to restore the blood glucose level of diabetic patients to a relatively stable state, and at the same time, providing personalized medication delivery, diet and exercise plans to facilitate the patients to achieve self-monitoring and management of blood glucose even after they have been discharged from the hospital. The device used to perform continuous glucose monitoring is typically a CGM, and the device used to perform drug infusion is typically an insulin pump. Because the inaccuracy of insulin infusion will cause a risk of Hyperglycemia and hypoglycemia to patients, the doctor must evaluate that the patients are physically able to use an insulin pump before using an insulin pump for insulin infusion, and the patient also need to follow the insulin infusion solution established by the doctor.

Currently when users conduct management via CGM and/or insulin pumps, a personal management device or a secondary management device is needed for management, and since some patients require a guardian to conduct management at the same time, both the patient and the guardian may need the personal management device or the secondary management device at the same time. In addition, in order to more fully conduct management, the patient or guardian may have multiple personal management devices or secondary management devices on hand at the same time to prevent one of them from running out of power or being occupied by other functions and thus not being able to fully conduct management in real time. However, when more devices are used in the system, they may be lost for different reasons.

Therefore, in the prior art, there is an urgent need for a method of locating a lost device and a method of preventing the device loss in a diabetes management system.

### BRIEF SUMMARY OF THE INVENTION

The embodiment of the present invention discloses a method of locating a lost device in a diabetes management system, the diabetes management system comprises at least one wearable medical device; at least one personal management device and one secondary management device, at least one of the personal management device and the secondary management device is configured to control the wearable medical device, the personal management device and the secondary management device are communicatively connected; when either the personal management device or the secondary management device is determined as lost, the other device is used to locate the lost one, thereby enabling the user to retrieve it with ease..

The invention discloses a method of locating a lost device in a diabetes management system, the diabetes management system comprising: at least one wearable medical device; at least one personal management device and one secondary management device, at least one of the personal management device and the secondary management device is configured to control the wearable medical device, the personal management device and the secondary management device are communicatively connected; when one of the personal management device and the secondary management device is determined as lost, locating the lost device using the other one of the personal management device and the secondary management device.

According to one aspect of the present invention, further comprises confirming a communication connection status in the personal management device and the secondary management device.

According to one aspect of the present invention, when the personal management device and the secondary management device are in a state of communication connection, an instruction is sent to the lost device via the non-lost device.

According to one aspect of the present invention, both the personal management device and the secondary management device are provided with a locator that sends its own location information to the other when the personal management device and the secondary management device are in a state of communication connection, and stores the location information of the other.

According to one aspect of the present invention, when the personal management device and the secondary management device are in an unconnected state locating the lost device based on the most recent location information of the lost device stored on the non-lost device.

According to one aspect of the present invention, gradually approaches the non-lost device to the most recent location and sends a request for a communication connection to the lost device, the personal management device and the secondary management device establish a communication connection when the personal management device and the secondary management device are within a distance of a communication connection.

According to one aspect of the present invention, when a communication connection is established between the personal management device and the secondary management device, the non-lost device automatically sends an instruction to the lost device.

According to one aspect of the present invention, the lost device provides a prompt to indicate the specific location of the lost device after receiving the instruction.

According to one aspect of the present invention, the prompt is one or a combination of audible prompts, vibration prompts, and color prompts.

According to one aspect of the present invention, when the personal management device or the secondary management device does not receive location information from the other within a predetermined time interval, the personal management device and/or the secondary management device issues an alarm.

According to one aspect of the present invention, the personal management device and the secondary management device periodically send their own location information to the other when they are in a communication connection state, the predetermined time interval is an integer multiple times of the time interval at which the personal management device and the secondary management device periodically send their own location information to the other.

According to one aspect of the present invention, the setting of the time interval for the personal management device and the secondary management device to periodically send their own location information to the other is associated with a communication method between the personal management device and the secondary management device.

According to one aspect of the present invention, the method of communication between the personal management device and the secondary management device comprises at least one or more of Bluetooth, mobile cellular, WiFi, Internet.

According to one aspect of the present invention, the alarm is one or a combination of audible alarms, vibration alarms, and interface text alarms.

According to one aspect of the present invention, a user further views the location or communication connection status of the personal management device and the secondary management device after receiving the alarm

According to one aspect of the present invention, the personal management device and/or the secondary management device issue an alarm when the personal management device and the secondary management device are disconnected from the communication connection.

The invention also discloses a method of preventing a device loss in a diabetes management system, the diabetes management system comprising: at least one wearable medical device; at least one personal management device and one secondary management device, at least one of the personal management device and the secondary management device is configured to control the wearable medical device; both the personal management device and the secondary management device are provided with a locator and sends its own location information to the other when the personal management device and the secondary management device are in a state of connection, and stores the other's location information; and the personal management device and/or the secondary management device issue an alarm when the personal management device or the secondary management device fails to receive the other's location information within a predetermined time interval, or when the personal management device and the secondary management device are disconnected from the communication connection.

According to one aspect of the present invention, the personal management device and the secondary management device periodically send their own location information to the other, the predetermined time interval is an integer multiple times of the time interval at which the personal management device and the secondary management device periodically send their own location information to the other.

Compared with the prior art, the technical solution of the present invention has the following advantages:
In the diabetes management system disclosed in the present invention, comprising at least one wearable medical device; at least one personal management device and a secondary management device, at least one of the personal management device and the secondary management device is configured to control the wearable medical device, the personal management device and the secondary management device are communicatively connected; when one of the personal management device and the secondary management device is determined as lost, the other device is used to locate the lost one, thereby enabling the user to retrieve it with ease.

Furthermore, when the personal management device and the secondary management device are in a connected state, instructions are sent to the lost device through the non-lost device, and the lost device receives the instructions and provides prompts to indicate the specific location of the lost device, which facilitates the user to quickly search for the lost device.

Furthermore, both the personal management device and the secondary management device are provided with a locator, when the personal management device and the secondary management device are in a communication connection state, transmits their own location information to the other and stores the location information of the other. When the personal management device and the secondary management device are in an unconnected state, the most recent location information of the lost device is reviewed on the non-lost device, so that on the one hand, the user can quickly retrieve the lost device directly based on the most recent location information, on the other hand, the user can gradually approach the non-lost device to the most recent location of the lost device and send a communication connection request to the lost device. When the personal management device and the secondary management device are within the distance of the communication connection, the personal management device and the secondary management device are switched from the unconnected state to the connected state, at which time the non-lost device automatically sends instructions to the lost device, and the lost device receives the instructions and provides a prompt to indicate the specific location of the lost device, which facilitates the user to retrieve the lost device more quickly.

Furthermore, when the personal management device or the secondary management device does not receive the location information from the other for a predetermined time period, the personal management device and/or the secondary management device sends out an alarm to remind the user that the device may be lost, and the user further checks the location or the connection status of the personal management device and the secondary management device after receiving the alarm, which can prevent the loss of the device.

Furthermore, when the personal management device and the secondary management device are disconnected, the personal management device and/or the secondary management device emits an alarm to alert the user that the connection between the personal management device and the secondary management device has been disconnected, prompting the re-establishment of the communication connection as soon as possible to prevent loss of the device.

Furthermore, the predetermined time interval in which the personal management device and/or the secondary management device does not receive the location information from the other is an integer multiple times of the time interval in which the personal management device and the secondary management device periodically send their own location information to the other when the personal management device and the secondary management device are in the connected state, when the time interval in which the two periodically send the location information to the other is longer, the multiple times is smaller, and when the time interval is shorter, the multiple times is larger. On the one hand, it is possible to avoid triggering unnecessary alarms when the personal management device and/or the secondary management device fails to send its own location information properly due to random or transient errors, on the other hand, avoid making it more difficult to search for a lost device due to the disconnection of the personal management device and the secondary management device.

Furthermore, the personal management device and the secondary management device include at least one of a dedicated handheld device, a smartphone, and a personal computer. The user of the personal management device and the secondary management device may be a patient and/or a guardian, and the device they used may be more than one, and the device they used may be the same or different, which is convenient for the user to make a choice according to the different needs, and improves the user experience.

### BRIEF DESCRIPTION OF THE DRAWINGS

FIG.1 is a schematic diagram of the glucose management system according to an embodiment of the present invention.
FIG.2 is a schematic diagram of the integrated continuous glucose monitoring device according to an embodiment of the present invention.
FIG.3 is a schematic diagram of the split continuous glucose monitoring device according to an embodiment of the present invention.
FIG.4a is a schematic diagram of the integrated insulin pump according to an embodiment of the present invention.
FIG.4b is a schematic diagram of the split insulin pump according to an embodiment of the present invention.
FIG.5 is a flowchart of a method of locating a lost device according to an embodiment of the present invention.

### DETAILED DESCRIPTION

As mentioned above, in order to meet different usage needs in different scenarios, multiple management devices may be used in the diabetes management system, and the devices may be lost due to various reasons, so there is a need for a method that can be used in the diabetes management system to locate the lost devices as soon as possible and to prevent the loss of the devices.

In order to solve this problem, the present invention provides a method of locating a lost device in a diabetes management system, the diabetes management system comprises at least one wearable medical device; at least one personal management device and a secondary management device, at least one of the personal management device and the secondary management device is configured to control the wearable medical device, the personal management device and the secondary management device are communicatively connected; when one of the personal management device and the secondary management device is determined as lost, the other device is used to locate the lost one, thereby enabling the user to retrieve it with ease..

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

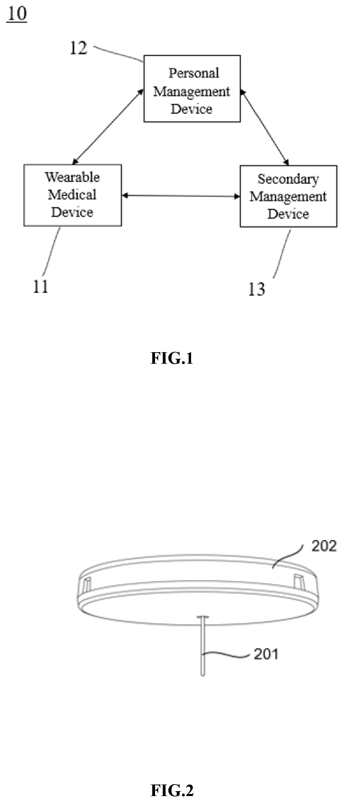

FIG.1 is a schematic diagram of the glucose management system.

In embodiments of the present invention, a blood management system 10 includes at least one wearable medical device 11, a personal management device 12 and a secondary management device 13.

The wearable medical device 11 comprising a blood glucose monitoring device (CGM) and/or an insulin pump, the blood glucose monitoring device is used for real-time monitoring of the patient's blood glucose level, mounted on the patient by means of an auxiliary mounting, comprising an implantable sensor, the sensor is connected to a transmitter, the transmitter further comprising a memory, a processor, a communication interface, and the like, the transmitter is used for transmitting information about blood glucose data monitored by the blood glucose monitoring device, as well as an identifier information of the blood glucose monitoring device, and the like. The insulin pump used by the patient contains an infusion mechanism and a control mechanism which is necessary for the infusion of insulin, the infusion mechanism includes a drug reservoir, a drive structure, an infusion fluid path and an infusion needle, a power supply, a circuit board, and the like, and the control mechanism includes a memory, a processor, a communication interface, and the like. The infusion mechanism is used to infuse insulin required by the patient in the patient's body, and the control mechanism is used to receive insulin infusion prompt and send insulin infusion status and insulin device identifier information and the like.

At least one of the personal management device 12 or the secondary management device 13 is used to control the operation of the medical device 11, both of them include a memory, a processor, a communication interface, a display, a user interface, and the like. Programming instructions are stored in the memory and the processor can execute the programming instructions in the memory. When both the personal management device 12 and the secondary management device 13 are used to control the medical device 11, such as the blood glucose monitoring device, after being mounted on the surface of the skin of the user, monitors the blood glucose concentration in the user's body and sends the blood glucose concentration information to the personal management device 12 and the secondary management device 13. The personal management device 12 and the secondary management device 13 receive the blood glucose concentration information and analyze and sort out it, and ultimately display it in an intuitive manner on a display screen of the personal management device 12 and the secondary management device 13. The insulin pump carries out insulin infusion according to the infusion instruction received from the personal management device 12 and/or the secondary management device 13, and at the same time feeds back the infusion status to the personal management device 12 and/or the secondary management device 13, and the personal management device 12 and the secondary management device 13 analyze and sort out the information of insulin infusion after receiving the information of insulin infusion, and ultimately display it on the display screen of the personal management device 12 and the secondary management device 13 in an intuitive manner.

When one of the personal management device 12 and the secondary management device 13 is used to control the operation of the medical device 11, the blood glucose monitoring device and the insulin pump send the blood glucose information and the insulin infusion information to the device controlling the medical device 11, such as the personal management device 12, as previously described, and the personal management device 12 displays the blood glucose information and the insulin infusion information in an intuitive manner, whereas the secondary management device 13, by communicating with the personal management device 12, can likewise view and display the blood glucose information and the insulin infusion information. Similarly, the blood glucose monitoring device and the insulin pump send the blood glucose information and the insulin infusion information to the secondary management device 13 in the manner as previously described, the secondary management device 13 displays the blood glucose information and the insulin infusion information in an intuitive manner, and the personal management device 12 is able to view and display the blood glucose information and the insulin infusion information through the secondary management device 13.

The personal management devices 12 and/or the secondary management devices 13 include dedicated handheld devices, smartphones that can download a dedicated app, personal computers with access to a specialized website, tablet PCs, and so on.

It is to be noted that in the embodiment of the present invention, the personal management device 12 and the secondary management device 13 may be the same device or different devices, and may be the same in function and use, therefore, the personal management device 12 and the secondary management device 13 are not divided into primary and secondary in function in the embodiment of the present invention, and are only named differently to distinguish the two different devices. In some embodiments of the present invention, the wearable medical device 11 is wirelessly communicatively connected to the personal management device 12 and/or the secondary management device 13 via Bluetooth, mobile cellular, Wi-Fi, or the Internet. In one embodiment of the present invention, the wearable medical device 11 is wirelessly communicatively connected to the personal management device 12 and/or the secondary management device 13 via Bluetooth, which can save the power consumption of the wearable medical device 11 and prolong its service life.

In some embodiments of the present invention, the personal management device 12 may establish a wireless communication connection with the secondary management device 13 by means of Bluetooth, Wi-Fi, or Internet, or establish a wired communication connection with the secondary management device 13 by means of USB, serial, Ethernet, or the like, in order to realize data transmission between the personal management device 12 and the secondary management device 13.

In some embodiments of the present invention, the diabetes management system 10 further comprises a remote server that communicates with the personal management device 12 and/or the secondary management device 13 via Wi-Fi or the Internet, etc., storing information uploaded by the personal management device 12 and/or the secondary management device 13, transmitting information to the personal management device 12 and/or the secondary management device 13, such as medical device's software or firmware update information, whether the pairing of the medical device 11 with the management device is successful or not, whether the medical device 11 is an already utilized device or whether it needs to be updated, etc., the specific method of updating the software or firmware of the medical device can reference to the PCT application: PCT/CN2023/114922, the entire contents of which are herein incorporated into the text by way of introduction into the full text.

The personal management device 12 and/or the secondary management device 13 may be the same device used simultaneously by the patient, the guardian, etc., or the patient and the guardian may use separate devices. Preferably, the personal management 12 device is a specialized handheld device (PDM), the secondary management device 13 is a smartphone, the wearable medical device 11 communicates with the PDM via Bluetooth, the PDM communicates with the smartphone via Bluetooth, and the wearable medical device 11 communicates with the smartphone via Bluetooth, mobile cellular, Wi-Fi, or Internet. When the patient and/or the guardian use the PDM and the smartphone at the same time, it is convenient for the user to choose to use the PDM and/or the smartphone according to the actual situation, such as switching to use the other to control the wearable medical device 11 when one of them is low on power, or when the cellular phone is being occupied by other functions such as calling, video recording, conferencing, and so on.

When the patient and the guardian use a device separately, such as the patient uses PDM and the guardian uses a smartphone, on the one hand, it allows the patient and the guardian to view the patient's blood glucose information and realize the monitoring of the medical equipment at the same time, on the other hand, the family does not need to purchase more equipment, to avoid the increase of unnecessary medical expenses. Especially when the patient is an elderly person or a young child, or a diabetic patient with a special disease who cannot use the management device for self-monitoring of blood glucose, the healthcare provider can turn on the locking mode through the smartphone to limit the patient's operation on the PDM, so that the patient cannot view the real-time blood glucose and/or insulin infusion data, receive blood glucose alarms, or change the alarm setting on the PDM, and so on, and it can prevent the patient's misoperation to the PDM, such as deleting the device, disconnecting the PDM and the medical device, changing the insulin infusion settings, which may affect the normal work of the medical device; not receiving the blood glucose alarm can also avoid the blood glucose alarm from interfering with or affecting the patient.

It is to be noted that in the embodiments of the present invention there is no limitation on the type and number of personal management devices and secondary management devices to be used by the patient and/or the guardian, i.e. the patient and/or the guardian may use one or more of the same or different devices for controlling the wearable medical device according to the actual need.

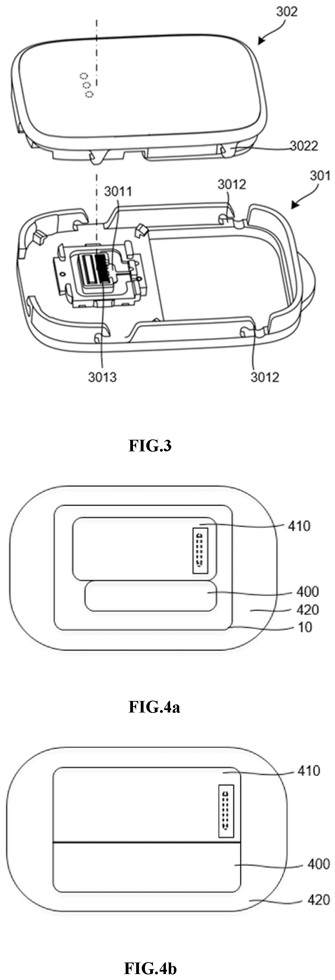

FIG.2 is a schematic diagram of the integrated continuous glucose monitoring device according to an embodiment of the present invention. FIG.3 is a schematic diagram of the split continuous glucose monitoring device according to an embodiment of the present invention.

The CGM comprising a sensor and a transmitter, mounted on the patient by means of an auxiliary mount, pierced subcutaneously. The sensor is used to collect analyte data in the human body and transmit the collected analyte content information. The transmitter is connected to the sensor, and it is used to receive the information on the blood glucose data, which is transmitted by the sensor that has been implanted under the skin, and convert the information into a wireless signal for outputting, and at the same time, it can also output other information such as information on an identifier of the CGM. Each CGM has a unique identifier including, but not limited to a device identifier, a hardware identifier, a generic unique identifier, a serial number, an identifier based on communication protocol (e.g., a BLE ID), a manufacturer's identifier, and the like, preferably, the identifier is formed by a plurality of randomly-combined numerical and alphabetic, and can be set on a housing or package of the CGM, or may also be set differently depending on the type of the CGM.

FIG.2 is a schematic diagram of the integrated continuous glucose monitoring device, i.e., the sensor and the transmitter have been integrated together prior to use, and is a single-use product, which is discarded after use, as shown in FIG. 2. The integrated continuous glucose monitoring device includes a sensor 201, a housing 202, and a transmitter (not shown in the figure) set inside the housing 202, the sensor 301 is used to monitor the patient's body fluid blood glucose data information, and transmit the said blood glucose data information to the transmitter through an internal circuit, which in turn sends it to a receiver. The identifier may be provided on the outer housing of the CGM or on the outer packaging or within the CGM.

FIG.3 is a schematic diagram of the split continuous glucose monitoring device, i.e., the sensor and the transmitter are two different parts prior to use, packaged separately, and only integrated together when in use, as shown in FIG. 3. The split continuous glucose monitoring device includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose data information, the blood glucose data information is transmitted to the transmitter 302 through the electrical connection 3013, and then sent to the receiver by the transmitter 302.

In one embodiment of the present invention, both the sensor and the transmitter of the split continuous glucose monitoring device are single-use products, which are discarded after use, and therefore the identifier may be provided on the housing or the outer packaging of the sensor or the transmitter. In yet another embodiment of the present invention, only the sensor of the split continuous glucose monitoring device is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

FIG.4a is a schematic diagram of the integrated insulin pump, i.e., the infusion mechanism 410 and the control mechanism 400 of the insulin pump are provided inside the same housing 10, and both are connected by wires and are affixed to a location on the user's skin by means of an adhesive patch 420, which is disposed of in its entirety after a single use; an identifier may be provided on the outer housing of the insulin pump or on the outer packaging or inside the insulin pump.

FIG.4b is a schematic diagram of the split insulin pump, i.e., the infusion mechanism 410 and the control mechanism 400 of the insulin pump are provided in two different housings, both of which are connected by waterproof plugs or directly snap together and electrically connected as a whole, an identifier may be provided on the outer housing of the infusion mechanism and/or the control mechanism or on the outer packaging or inside the insulin pump.

In one embodiment of the present invention, both the infusion mechanism and the control mechanism of the split insulin pump are single-use products, which are discarded after use, and therefore the identifiers may be provided on the housing or on the outer packaging of the infusion mechanism and/or the control mechanism. In yet another embodiment of the present invention, only the infusion mechanism of the split insulin pump is a single-use product, while the control mechanism is a reusable product, and therefore, preferably, in this embodiment, the identifier is set on the housing of the control mechanism or on the outer packaging, which can reduce the frequency of binding of patient information and identifiers, and improve the patient experience. This is described in more detail below.

When the identifier is set on the housing of the control mechanism or on the outer packaging, it is set in a form that includes, but is not limited to, a QR code, a bar code, or an NFC tag.

The CGM and/or insulin pump needs to be properly amounted to the skin before it can begin to work, and the patient's personal information is paired with information about the CGM and/or insulin pump to enable pairing of the management device with the patient. The patient's personal information includes name, age, gender, cell phone number, etc., and the information of the CGM and/or insulin pump being worn by the patient includes identifier information of the CGM and/or insulin pump. In one embodiment of the present invention, the PDM and the smartphone are respectively paired with the medical device to realize the control of the medical device, and further, the cell phone can also be paired with the PDM; in another embodiment of the present invention, the PDM is first paired with the medical device to realize the control of the medical device, and the smartphone is further paired with the PDM. At the same time the smartphone uploads the patient's personal information and the identifier information of the CGM and/or the insulin pump to a remote server, which may store the information uploaded by the smartphone and verify that the identifier information of the CGM and/or the insulin pump is valid, and if a certain identifier information already exists in the remote server, the remote server sends an alert to the smartphone to remind the user that the CGM or the insulin pump has already been used and needs to be replaced again. When the CGM and/or the insulin pump is mounted on the skin of the patient and successfully activated, the CGM and/or the insulin pump starts to work, the transmitter of the CGM sends the monitored blood glucose information to the smartphone either directly or via PDM, and further uploads it to the remote server, and the control mechanism of the insulin pump receives the insulin infusion information and controls the infusion mechanism to infuse the insulin, and at the same time sends the infusion status to the smartphone, and further uploads it to the remote server.

It is to be noted that the CGM and insulin pump in the embodiment of the present invention are developed and produced by the same manufacturer, and thus can be controlled by the same dedicated APP in the smartphone, or the same PDM so that when the patient needs both the CGM and the insulin pump at the same time, even if it is assumed that CGM or insulin pump produced by different manufacturers can be realized to directly control CGM or insulin pump by dedicated APP, which will inevitably cause inconvenience to users by using different APPs to control CGM and insulin pump separately; when users control CGM and insulin pump separately through PDM, as different manufacturers are equipped with dedicated PDM, users will inevitably need two PDM to control CGM and insulin pumps separately, which will affect the patient's user experience.

When the CGM and/or insulin pump worn by the patient needs to be replaced for reasons such as reaching the end of its life cycle or failing, the unique identifier information of the new CGM and/or insulin pump also needs to be updated by the management device by pairing it with the patient's personal information and further uploaded to a remote server via the smartphone, wherein the patient's personal information is entered manually, and the identifier information of the CGM and/or insulin pump may also be entered manually or by scanning a QR code, a barcode, or NFC tags on the housing or on the outer packaging of the CGM and/or the insulin pump.

When the CGM is split and the transmitter is reusable, the identifier of the CGM is set on the housing or packaging of the transmitter, so that the patient only needs to replace the sensor when replacing the CGM without replacing the transmitter, and the identifier of the CGM remains unchanged, and thus there is no need for updating the pairing update of the identifier of the CGM with the personal information of the patient via a smartphone or uploading it to a remote server anymore, and thus the number of operational steps can be reduced to improve the patient experience.

When the insulin pump is split and the control mechanism is reusable, the identifier of the insulin pump is set on the housing or packaging of the control mechanism, and the patient only needs to change the infusion mechanism when replacing the insulin pump without changing the control mechanism, and the identifier of the insulin pump remains unchanged, and thus there is no need for updating the pairing update of the identifier of the insulin pump with the personal information of the patient via the smartphone, and there is no need for uploading it to the remote server anymore, and thus the operational steps can be reduced and the patient experience can be improved.

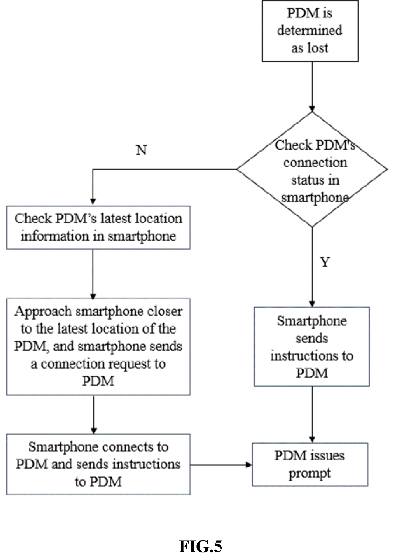

As previously mentioned, when a patient or a guardian uses a PDM and/or a smartphone at the same time, or when a patient and a guardian use a smartphone and a PDM separately, the smartphone or the PDM may be lost for one reason or another. Therefore, in the embodiment of the present invention, a locator is provided in the PDM, and the smartphone has a locator in the smartphone itself, and when the PDM and the smartphone are in a state of communication connection, the PDM and the smartphone send their own positional information to each other, and at the same time record each other's positional information, and the PDM and the smartphone send their own positional information to each other according to a predetermined rule, and preferably, both periodically send their own location information to the other and record the other's location information. When the user is unable to locate the PDM, as shown in FIG. 5, the user can check the communication connection status with the PDM on the smartphone, if the status is connected, an instruction will be sent to the PDM through the smartphone, and the PDM will provide prompt to instruct the user the specific location of the PDM after receiving the instruction. The prompt can be set as one or a combination of sound prompt, vibration prompt, and color prompt. The sound type, volume size, duration of the sound, frequency interval of the sound prompt; the vibration type, intensity of the vibration, duration of the vibration, frequency interval of the vibration prompt; the color type, change method and duration time of color prompt etc., which can be personalized according to the actual needs or the patient's preferences.

It should be noted that the device lost mentioned here may be that the user has temporarily forgotten the location of the smartphone or PDM, but the smartphone and the PDM are still in a communication connection state, and they still periodically transmit their own location information to the other and record the other's location information at the same time; the device lost may also be that the user not only forgets the location of the smartphone or the PDM, but also the communication connection between the smartphone and the PDM is disconnected because the actual distance exceeds the maximum distance for the two to communicate, so that the smartphone and the PDM are no longer periodically transmitting their own location information to other and recording the other's location information.

When the status of the communication connection between the smartphone and the PDM is disconnected, the user can review the location information of the PDM stored on the smartphone and determine the location of the PDM at the lost time based on the most recent location information stored on the smartphone, so that the PDM can be quickly located. In another embodiment of the present invention, as shown in FIG. 5, when the connection state of the smartphone and the PDM is disconnected, the user can check the most recent position information of the PDM stored on the smartphone and take the most recent position information of the PDM stored on the smartphone as the destination and gradually approach the smartphone towards the destination while sending a request for a communication connection to the PDM, the smartphone will be automatically connected to the PDM when the smartphone is within the communication connection distance of the PDM, and when the smartphone establishes a communication connection with the PDM, the smartphone automatically sends an instruction to the PDM, and the PDM, upon receipt of the instruction, will provide a prompt to instruct the user of the specific location of the PDM, in the specific manner of the prompt, as previously described. According to the prompt of PDM, the user can retrieve the PDM more quickly.

Similarly, if the user locates that the smartphone is lost, the user can adopt the same operation to locate the lost smartphone, i.e., the user can check the communication connection status with the smartphone on the PDM, and if the status is connected, the user can send an instruction to the smartphone through the PDM, and upon receipt of the instruction sent by the smartphone, the smartphone will provide a prompt to indicate the specific location of the user's smartphone; If the communication connection status between the smartphone and the PDM is disconnected, the user can check the latest position information of the smartphone stored on the PDM and use the latest position of the smartphone stored on the PDM as the destination to directly locate the lost smartphone at the destination, or gradually move the position of the PDM closer to the destination and at the same time send a communication connection request to the smartphone, so that the smartphone automatically connects with the PDM when the smartphone is within the communication connection distance of the PDM, and when the smartphone establishes a communication connection with the PDM, the PDM automatically sends an instruction to the smartphone, and the smartphone receives the instruction and provides a prompt to indicate the specific location of the user's smartphone, in a specific indication manner as previously described.

When the personal management device and the secondary management device used by the user are the same device, it is also possible to search for the lost device in the same way as described previously, and will not be repeated here.

In an embodiment of the present invention, when at least one of the personal management device and the secondary management is a PDM, the PDM communicates with the other device via Bluetooth, and due to the relatively short communication distance of Bluetooth, the frequency at which the personal management device and the secondary management device periodically transmit their own positional information to the other when they are in a connected state may be, for example, once every 5 minutes, once every 2 minutes, once every 1 minute, once every 30 seconds, once every 10 seconds, even once every 1 second, or at any specified frequency between once every 1 second and once every 5 minutes. When both the personal management device and the secondary management device are smartphones, since smartphones can communicate with each other via mobile cellular, WiFi or internet, the communication distance is relatively long, and in general, it is not easy to disconnect the communication connection between the two, the frequency of periodically sending its own location information to the other when the personal management device and the secondary management device are in the connection state may be set longer, such as once every 10 minutes, once every 30 minutes, or even once every an hour. Of course, the embodiment of the present invention does not limit the frequency with which the personal management device and the secondary management device send their own location information to the other, and the user can set the frequency according to the actual needs.

In one embodiment of the present invention, when the personal management device and/or the secondary management device do not receive the location information from the other within a predetermined time interval, the personal management device and/or the secondary management device sends out an alert to remind the user that the personal management device and/or the secondary management device may be lost, and the user is requested to check the location and the connection status of the personal management device and the secondary management device as soon as possible, so as to prevent the device lost.

If the personal management device or the secondary management device is lost, it can be searched for in the first instance as described previously; if the connection between the personal management device and the secondary management device is disconnected, a communication connection is established again to prevent the user taking the position of the personal management device or the secondary management device away from the most recently recorded position under the disconnection between the personal management device and the secondary management device, thus increasing the difficulty of searching for the personal management device or the secondary management device. The type of alarm can be set to one or a combination of sound, vibration, and interface text. The sound type, volume size, duration of the sound, frequency interval of the sound alarm; the vibration type, intensity of the vibration, duration of the vibration, frequency interval of the vibration alarm; the type of the interface text alarm, the frequency of the alarm, etc. can be personalized according to the actual needs or the patient's preferences.

In an embodiment of the present invention, the predetermined time interval in which the personal management device and/or the secondary management device do not receive the location information from the other within is an integer multiple times of the time interval in which the personal management device and the secondary management device periodically transmit their own location information to the other when the personal management device and the secondary management device are in the state of communication connection, e.g., when the interval in which the two parties periodically transmit the location information is relatively long, the predetermined time interval can be equal to the time interval in which they periodically send the location information. Under this condition, if the predetermined time interval is too long, the risk of the personal management device and the secondary management device disconnecting increases, and the risk of the lost device being taken away from the last stored location also increases, thereby increasing the difficulty of locating the lost device. When the time interval between the two periodically transmit the location information is relatively short, then the predetermined time interval can be equal to a majority of times of the time interval between the two periodically transmit the location information, such as 2-100 times. At this point, on the one hand, it is possible to avoid triggering unnecessary alarms when the personal management device and/or the secondary management device fails to transmit its own location information properly due to random or short-lived errors, while on the other hand, avoiding making it more difficult to search for a lost device due to disconnection of the personal management device and the secondary management device.

In another embodiment of the present invention, when the personal management device and/or the secondary management device is disconnected, the personal management device and/or the secondary management device emits an alert to alert the user that the connection between the personal management device and/or the secondary management device has been disconnected, prompting the re-establishment of the communication connection as soon as possible to prevent loss of the device.

In summary, the present invention discloses a method of locating a lost device in a diabetes management system, the diabetes management system comprises at least one wearable medical device; at least one personal management device and one secondary management device, at least one of the personal management device and the secondary management device is configured to control the wearable medical device, the personal management device and the secondary management device is communicatively connected; when one of the personal management device and the secondary management device is determined as lost, the other device is used to locate the lost one, thereby enabling the user to retrieve it with ease.

While the invention has been described in detail with reference to the specific embodiments of the present invention, it should be understood that it will be appreciated by those skilled in the art that the above embodiments may be modified without departing from the scope and spirit of the invention. The scope of the invention is defined by the appended claims.

## Claims

1. A method of locating a lost device in a diabetes management system, the diabetes management system comprising:
at least one wearable medical device;
at least one personal management device and one secondary management device, wherein at least one of the personal management device and the secondary management device is configured to control the wearable medical device, the personal management device and the secondary management device are communicatively connected; wherein
when one of the personal management device and the secondary management device is determined as lost, locating the lost device using the other one of the personal management device and the secondary management device.

2. The method of locating a lost device in a diabetes management system of claim 1, wherein,
further comprises confirming a communication connection status in the personal management device and the secondary management device.

3. The method of locating a lost device in a diabetes management system of claim 2, wherein,
when the personal management device and the secondary management device are in a state of communication connection, an instruction is sent to the lost device via the non-lost device.

4. The method of locating a lost device in a diabetes management system of claim 2, wherein,
both the personal management device and the secondary management device are provided with a locator that sends its own location information to the other when the personal management device and the secondary management device are in a state of communication connection, and stores the location information of the other.

5. The method of locating a lost device in a diabetes management system of claim 4, wherein,
when the personal management device and the secondary management device are in an unconnected state, locating the lost device based on the most recent location information of the lost device stored on the non-lost device.

6. The method of locating a lost device in a diabetes management system of claim 5, wherein,
gradually approaches the non-lost device to the most recent location and sends a request for a communication connection to the lost device, the personal management device and the secondary management device establish a communication connection when the personal management device and the secondary management device are within a distance of a communication connection.

7. The method of locating a lost device in a diabetes management system of claim 6, wherein,
when a communication connection is established between the personal management device and the secondary management device, the non-lost device automatically sends an instruction to the lost device.

8. The method of locating a lost device in a diabetes management system of claim 3 or 6, wherein,
the lost device provides a prompt to indicate the specific location of the lost device after receiving the instruction.

9. The method of locating a lost device in a diabetes management system of claim 8, wherein,
the prompt is one or a combination of audible prompts, vibration prompts, and color prompts.

10. The method of locating a lost device in a diabetes management system of claim 4, wherein,
when the personal management device or the secondary management device does not receive location information from the other within a predetermined time interval, the personal management device and/or the secondary management device issues an alarm.

11. The method of locating a lost device in a diabetes management system of claim 10, wherein,
the personal management device and the secondary management device periodically send their own location information to the other when they are in a communication connection state, the predetermined time interval is an integer multiple times of the time interval at which the personal management device and the secondary management device periodically send their own location information to the other.

12. The method of locating a lost device in a diabetes management system of claim 11, wherein,
the setting of the time interval for the personal management device and the secondary management device to periodically send their own location information to the other is associated with a communication method between the personal management device and the secondary management device.

13. The method of locating a lost device in a diabetes management system of claim 12, wherein,
the method of communication between the personal management device and the secondary management device comprises at least one or more of Bluetooth, mobile cellular, WiFi, Internet.

14. The method of locating a lost device in a diabetes management system of claim 10, wherein,
the alarm is one or a combination of audible alarms, vibration alarms, and interface text alarms.

15. The method of locating a lost device in a diabetes management system of claim 14, wherein,
a user further views the location or communication connection status of the personal management device and the secondary management device after receiving the alarm.

16. The method of locating a lost device in a diabetes management system of claim 1, wherein,
the personal management device and/or the secondary management device issue an alarm when the personal management device and the secondary management device are disconnected from the communication connection.

17. A method of preventing a device loss in a diabetes management system, the diabetes management system comprising:
at least one wearable medical device;
at least one personal management device and one secondary management device, wherein at least one of the personal management device and the secondary management device is configured to control the wearable medical device; wherein
both the personal management device and the secondary management device are provided with a locator and sends its own location information to the other when the personal management device and the secondary management device are in a state of connection, and stores the other's location information; and the personal management device and/or the secondary management device issue an alarm when the personal management device or the secondary management device fails to receive the other's location information within a predetermined time interval, or when the personal management device and the secondary management device are disconnected from the communication connection.

18. The method of preventing a device loss in a diabetes management system of claim 17, wherein,
the personal management device and the secondary management device periodically send their own location information to the other, the predetermined time interval is an integer multiple times of the time interval at which the personal management device and the secondary management device periodically send their own location information to the other.
